# EUROPEAN PATENT APPLICATION

(11) **EP 0 877 020 A1**
(43) Date of publication of application: **11.11.1998**
(21) Application number: 95932954.1
(22) Date of filing: 02.10.1995
(51) Int. Cl.: C07D 219/06, C07D 401/06, C07D 405/06, C07D 409/06, A61K 31/435, A61K 31/495, A61K 31/535, A61K 31/445, A61K 31/44

(54) **ACRIDONE DERIVATIVE**

(71) Applicant: Eisai Co., Ltd., Tokyo 112-88 (JP)
(72) Inventor: MIYAMOTO, Mitsuaki, Tsuchiura-shi, Ibaraki 300 (JP); YOSHIUCHI, Tatsuya, Tsukuba-shi, Ibaraki 305 (JP); ABE, Shinya, Ushiku-shi, Ibaraki 300-12 (JP); TANAKA, Masayuki, Ushiku-shi, Ibaraki 300-12 (JP); MORIYA, Katsuhiro, Tsukuba-shi, Ibaraki 305 (JP); KATAYAMA, Satoshi, Tsukuba-shi, Ibaraki 305 (JP); YAMANAKA, Takashi, Tsukuba-shi, Ibaraki 305 (JP); YAMADA, Koji, Ushiku-shi, Ibaraki 300-12 (JP)
(74) Representative: Hansen, Bernd, Dr. Dipl.-Chem.
(86) International application number: JP9502007
(87) International publication number: WO9712871

(57) **Abstract**

An acridone derivative represented by the following formula (I) and a pharmacologically acceptable salt thereof are effective in preventing and curing diseases which are associable with histamine, leucotriene, etc. and in preventing and curing allergic diseases such as asthma, allergic rhinitis, atopic dermatitis, urticaria, valerian fever, digestive tract allergy, or food allergy. 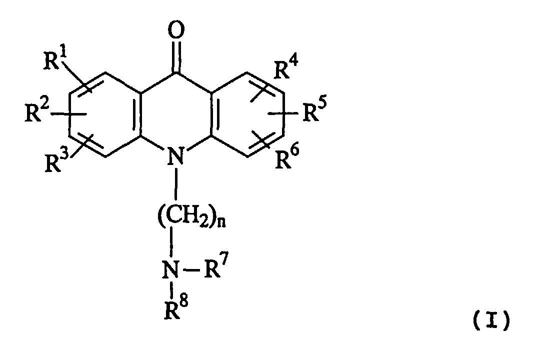 wherein R¹ - R⁸ severally represent a hydrogen atom, a lower alkyl group, etc. and n represents an integer of 1 - 6.

## Description

### BACKGROUND OF THE INVENTION

### Field of the Invention

This invention relates to a novel acridone derivative and/or a pharmacologically acceptable salt thereof which is effective in the preventive therapy of diseases associable with histamine, leucotriene, prostaglandin, TNF, etc.

### Description of Related Art

The bronchial asthma and the atopic diseases in human beings appear in consequence of highly intricate vital reactions. It is suspected that most of these conditions are caused because various chemically infectious substances liberated from mast cells and basophils, as triggered by antigen-antibody reactions, induce vital disturbances as by contracting such smooth muscles as bronchial muscles and vessels of the pulmonary circulation or enlarging peripheral vessels in terms of blood passage.

As the chemically infectious substances liberated from mast cells and basophils, histamine, leucotrienes, prostaglandins, TNF, etc. have been known. It is well known that histamine, among other substances mentioned above, is the most significant chemically infectious substance for the allergic rhinitis and the urticaria in human beings. The leucotrienes comprise leucotrienes B₄, C₄, and D₄ and the relation thereof with the asthmatic convulsion has been attracting attention.

Heretofore, the development of medicines for the prevention, alleviation, or elimination of the crisis of symptoms of allergic diseases has been aimed at repressing the creation and liberation of such chemically infectious substances or antagonizing the effects thereof.

The medicines of this sort are represented by sodium cromoglycate (Intal™) which was introduced to the market in 1969.

The conventional antiallergic agents which are represented by Intal™, however, encounter discrepancy between the concentration for in vitro repression of the liberation of chemically infectious substances and the concentration for in vivo repression of the liberation and involve numerous ambiguous points concerning mechanism. Thus, the development of an antiallergic agent of a new type capable of manifesting a further improved clinical effect.

### DISCLOSURE OF THE INVENTION

### Summary of the Invention

An object of this invention, therefore, is to provide a novel acridone derivative and a pharmacologically acceptable salt thereof which are effective as an antiasthmatic agent and an antiallergic agent (the preventive and curative agents for allergic rhinitis, atopic dermatitis, urticaria, digestive tract allergy, and food allergy). Another object of this invention is to provide a medicine having as an active component thereof the compound and/or a pharmacologically acceptable salt thereof.

The mast cell and the basophil which concern the allergic diseases in large measure are possessed on the cell membrane thereof of the receptor FcεRI having high affinity for the IgE antibody. The IgE antibody bound to this receptor, owing solely to the cross-linkage thereof with a corresponding polyvalent antibody, activates the signal transmitting mechanism in the cell. It is inferred that the activation of the intracellular signal transmission mechanism induces the liberation of histamine or the formation and liberation of prostanoids like leucotrienes and prostaglandins and terminates in the manifestation of the so-called allergic symptoms. It is further inferred that the resultant cytokines such as TNF and interleukins participate in the impartation of chronicity to the relevant diseases via the interaction thereof with other cells.

The present inventors, in view of this true state of affairs, have come to take particular notice of the stage of activation of the non-receptor type thyrosine kinase which occupies the initial phase of the activation of the intracellular signal transmission mechanism among other component phases of the mechanism for the liberation of a chemically infectious substance from the mast cell and the basophil. It has been known that the thyrosine kinase effects this activation by binding itself with the phosphorylated thyrosine activating motif area on the IgE receptor γ chain. By inhibiting this bondage, therefore, the activation of the intracellular signal transmitting mechanism by the IgE antibody-dependent mast cell and basophil and, consequently, the liberation of the chemically infectious substances mentioned above can be inhibited. The present inventors, therefore, have continued a diligent study with a view to developing an antiallergic agent based on the entirely new mechanism of inhibiting the bondage of the thyrosine kinase with the thyrosine activating motif area. They have been consequently ascertained that the following acridone derivative accomplishes the objects of this invention. The present invention has been perfected based on this knowledge.

Specifically, this invention is directed to providing an acridone derivative represented by the following formula (I) or a pharmacologically acceptable salt thereof:

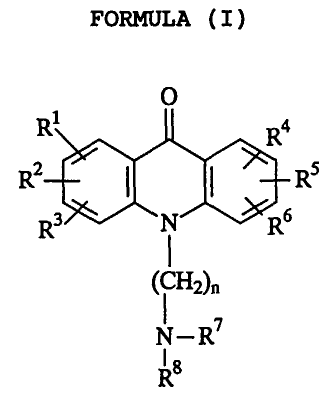

In the formula, R¹, R², R³, R⁴, R⁵, and R⁶ invariably or variably represent a hydrogen atom, a lower alkyl group, a lower alkoxy group, or a halogen atom, R⁷ and R⁸ invariably or variably represent a hydrogen atom, a lower alkyl group, an aryl group, a heteroaryl group, an aralkyl group, or a heteroaryl alkyl group, providing R⁷ and R⁸, when taken together with a nitrogen atom to which they are bound, form a ring of five or six members, wherein the ring optionally possesses as component atoms thereof an oxygen atom or a sulfur atom or a group represented by the formula: >N-R⁹ (wherein R⁹ represents a hydrogen atom, a lower alkyl group, an aryl group, or an aralkyl group) in addition to carbon atoms, and n represents an integer in the range of 1 - 6.

In the acridone derivatives which are represented by the formula (I) mentioned above or the pharmacologically acceptable salts thereof, those acridone derivatives answering the formula (I) by having a hydrogen atom for R⁴ and a lower alkyl group, a lower alkoxy group, or a halogen atom each for R⁵ and R⁶ and having these substituents attached respectively to the 3 position and the 4 position and the pharmacologically acceptable salts thereof prove to be particularly advantageous.

This invention also provides a preventive·curative agent for the diseases in which the thyrosine kinase inhibiting action is effective, which agent has as an active component thereof an acridone derivative represented by the formula (I) and/or a pharmacologically acceptable salt thereof.

Further, this invention provides a preventive·curative agent for allergic diseases, which agent has as an active component thereof an acridone derivative represented by the formula (I) and/or a pharmacologically acceptable salt thereof.

The invention will be described specifically below.

### DETAILED DESCRIPTION OF THE INVENTION

The term "lower alkyl group" which is found in the definitions of R¹, R², R³, R⁴, R⁵, R⁶, R⁷, R⁸, and R⁹ used in the formula (I) mentioned above means Linear or branched alkyl groups of 1 - 8 carbon atoms such as, for example, methyl group, ethyl group, propyl group, isopropyl group, butyl group, isobutyl group, sec-butyl group, tert-butyl group, pentyl group (amyl group), isopentyl group, neopentyl group, tert-pentyl group, 1-methylbutyl group, 2-methylbutyl group, 3-methylbutyl group, 1,2-dimethylpropyl group, hexyl group, isohexyl group, 1-methylpentyl group, 2-methylpentyl group, 3-methylpentyl group, 1,1-dimethyl-butyl group, 1,2-dimethylbutyl group, 2,2-dimethylbutyl group, 1,3-dimethylbutyl group, 2,3-dimethylbutyl group, 3,3-dimethyl-butyl group, 2-ethylbutyl group, 2-ethylbutyl group, 1,1,2-trimethylpropyl group, 1,2,2-trimethylpropyl group, 1-ethyl-1-methylpropyl group, 1-ethyl-2-methylpropyl group, heptyl group, and octyl group.

The term "lower alkoxy group" which is found in the definitions of R¹, R², R³, R⁴, R⁵, and R⁶ used in the formula (I) mentioned above means linear or branched alkoxy groups of 1 - 8 carbon atoms such as, for example, methoxy group, ethoxy group, propoxy group, isopropoxy group, butoxy group, isobutoxy group, sec-butoxy group, tert-butoxy group, pentyloxy group (amyloxy group), isopentyloxy group, neopentyloxy group, tert-pentyloxy group, 1-methylbutoxy group, 2-methylbutoxy group, 3-methylbutoxy group, 1,2-dimethylpropoxy group, hexyloxy group, isohexyloxy group, 1-methylpentyloxy group, 2-methylpentyloxy group, 3-methylpentyloxy group, 1,1-dimethylbutoxy group, 1,2-dimethyl-butoxy group, 2,2-dimethylbutoxy group, 1,3-dimethylbutoxy group, 2,3-dimethylbutoxy group, 3,3-dimethylbutoxy group, 1-ethylbutoxy group, 2-ethylbutoxy group, 1,1,2-trimethylpropoxy group, 1,2,2-trimethylpropoxy group, 1-ethyl-1-methylpropoxy group, 1-ethyl-2-methylpropoxy group, heptyloxy group, and octyloxy group.

The term "halogen atom" means fluorine atom, chlorine atom, bromine atom, and iodine atom.

The term "aryl group" which is found in the definitions of R⁷, R⁸, and R⁹ used in the formula (I) mentioned above means phenyl group, biphenyl group, naphthyl group, anthryl group, and phenanthryl group and the term "heteroaryl group" means unsaturated 5- to 7-member rings containing one or two hetero atoms selected from the class consisting of oxygen atom, nitrogen atom, and sulfur atom in addition to carbon atoms. These aryl groups and heteroaryl groups are preferred not to contain any substituent. The term "aralkyl group" means benzyl group, phenylethyl group, 1-naphthylmethyl group, 2-naphthylmethyl group, 1-naphthylethyl group, and 2-naphthylethyl group, which are preferred not to contain any substituent. The part "heteroaryl" in the term "heteroarylalkyl group" has the same meaning as the term "heteroaryl group" mentioned above. In this case, the part "alkyl" has the same meaning as the term "lower alkyl group" mentioned above.

The ring which is formed by R⁷ and R⁸ jointly with the nitrogen atom with which the substituents are bound is a saturated or unsaturated 5- or 6-member ring optionally containing an oxygen atom, a sulfur atom, and/or a nitrogen atom as hetero atoms.

As concrete examples of the pharmacologically acceptable salt in this invention, inorganic salts such as hydrochlorides, hydrobromides, sulfates, and phosphates, organic salts such as acetates, maleates, tartrates, methane sulfonates, benzene sulfonates, and toluene sulfonates, and salts of such amino acids as aspartic acid and glutamic acid may be cited.

The compounds to which this invention pertains are useful as a preventive·curative agents for the diseases in which the thyrosine kinase inhibiting action is effective, specifically as preventive·curative agents for such allergic diseases as asthma, allergic rhinitis, atopic dermatitis, urticaria, valerian fever, digestive tract allergy, and food allergy. When they are used as preventive·curative agents for these diseases, the dosages thereof, though variable copiously with the kind of disease, the intensity of symptom, and the age of a patient, for example, generally fall in the approximate range of 0.03 - 100 mg, preferably 0.1 - 500 mg, and more preferably 0.1 - 100 mg, per day. These daily doses are used wholly once or portionwise several times.

These compounds contemplated by this invention can be administered in the form of tablet, powder, granule, capsule, syrup, inhaler, or injection. The medicines containing the compounds of this invention can be formulated in these forms by standard methods.

### [Method of production]

The compounds of this invention can be produced by known methods with necessary modifications. Typical methods available for the production of the compounds of this invention is shown below.

The compounds satisfying the formula (I) mentioned above by having a hydrogen atom severally for R¹ - R⁴, a methyl group severally for R⁵ and R⁶ respectively at the 3 position and the 4 position can be produced by a procedure consisting of Steps 1 - 5 and the compounds having no hydrogen atom for either or both of R⁷ and R⁸ by a procedure consisting of Steps 1 - 5 and Step 7 or a procedure consisting of Steps 1 - 3 and Step 6.

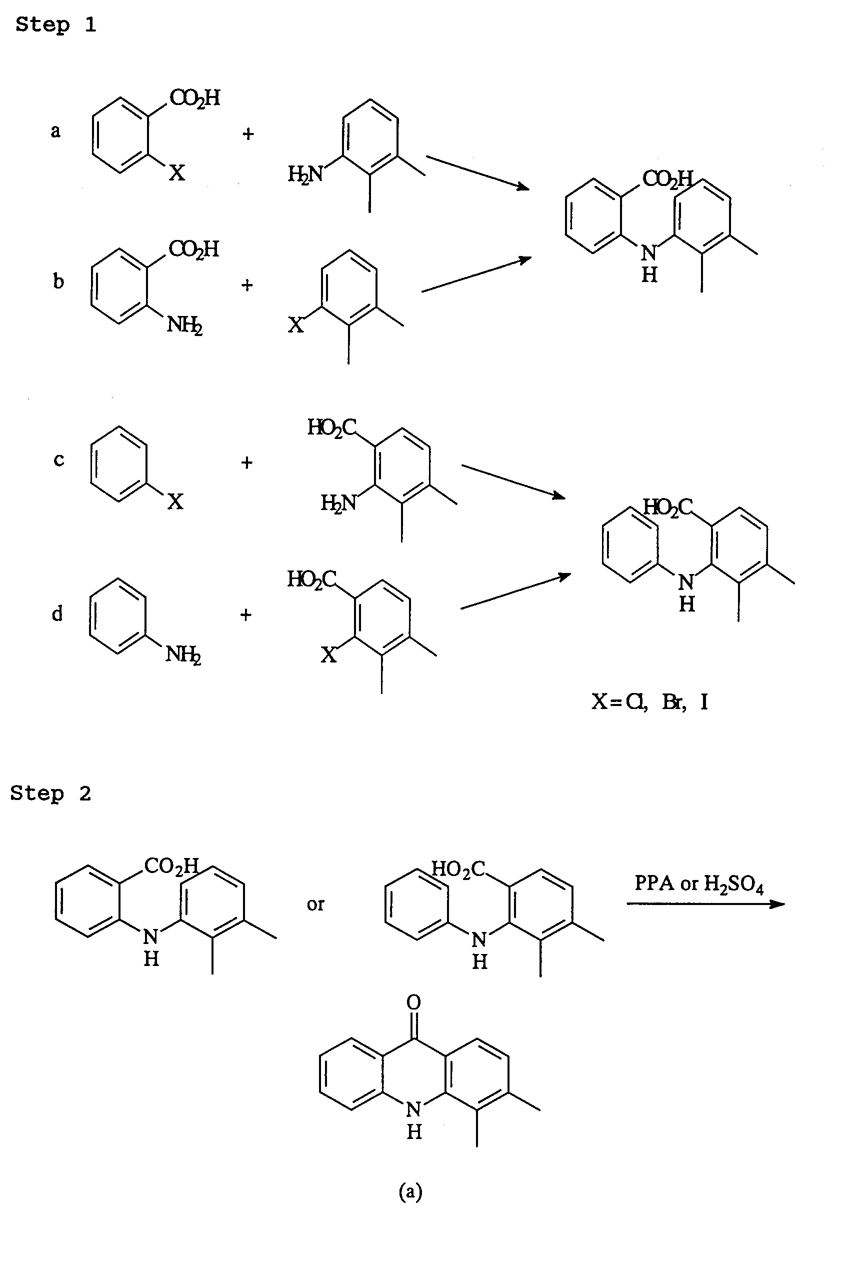

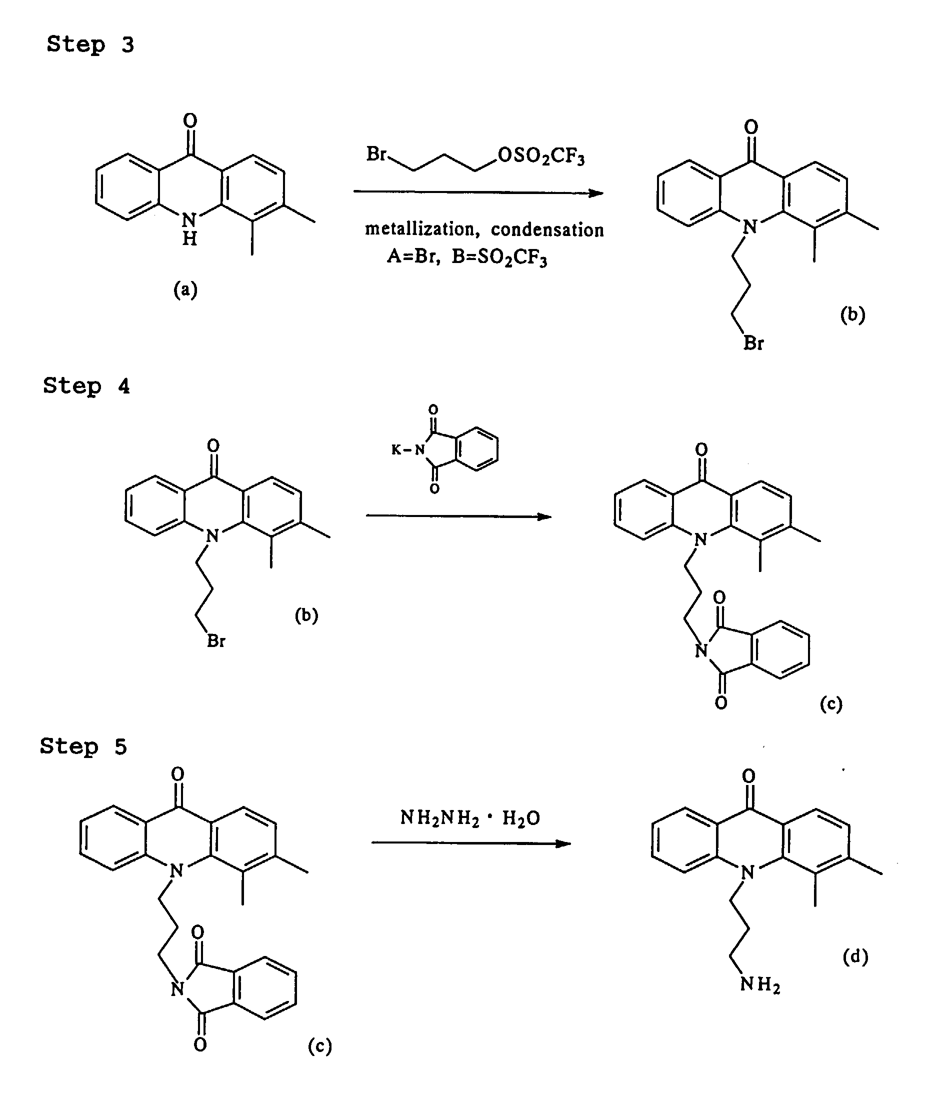

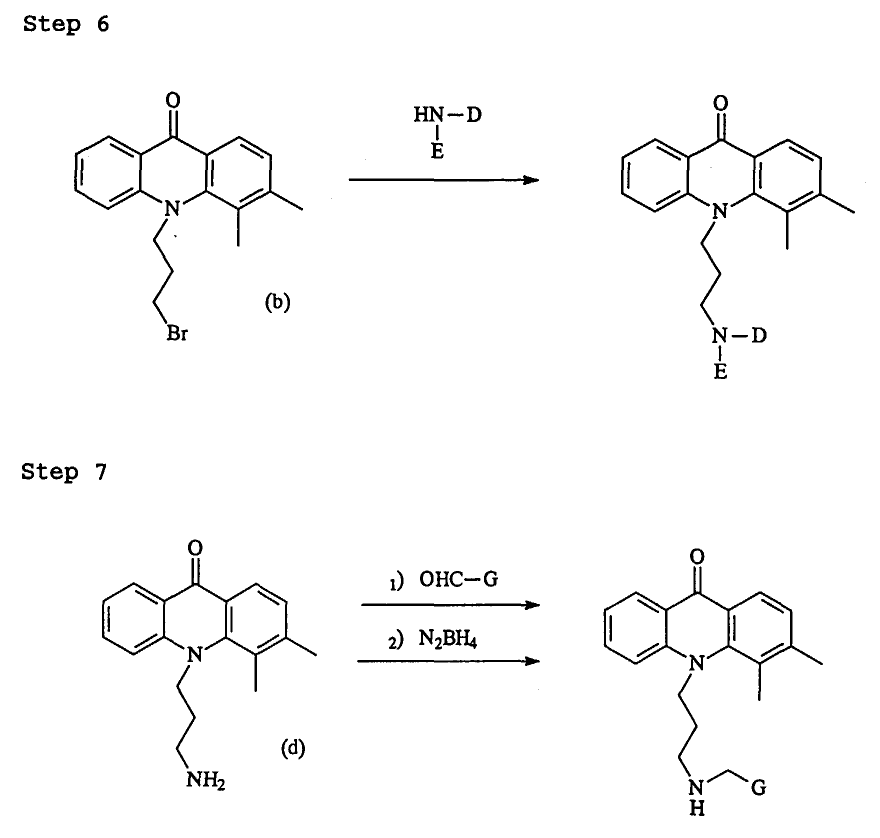

### Step 1

The necessary diphenyl amine can be obtained by the Ullmann reaction in any of the reactions a - d. Specifically, this step comprises solving a mixture of an amine with a halide in dimethyl formamide, adding potassium carbonate and a catalytic amount of copper powder to the resultant solution, and heating the mixed solution at an elevated temperature. As the catalyst, cuprous iodide may be used instead of the copper powder. As the solvent, amyl alcohol may be used instead of the dimethyl formamide.

### Step 2

The formation of acridone [Compound (a)] by the cyclization of diphenyl amine is performed by using polyphosphoric acid or sulfuric acid as a solvent at a temperature in the range of from room temperature to about 80°C.

### Step 3

The compound (b) is prepared by metallizing the nitrogen atom of the acridone ring of the compound (a) and condensing the resultant compound with an aliphatic saturated hydrocarbon having liberating groups A and B severally at the opposite terminals thereof.

For this metallization, metal alkyls such as butyl lithium, metal-containing amines such as lithium diisopropyl amine and lithium bis(trimethyl silyl) amide, and metal hydrides such as sodium hydride and potassium hydride are available. The solvent to be used in the metallization and the condensation may be suitably selected from among such solvents as diethyl ether, tetrahydrofuran and n-hexane which do not participate in the relevant reactions. The reaction temperature generally falls in the range of from -20°C to the refluxing point of the solvent. As concrete examples of the liberating groups A and B, halogen atoms, mesyl group, tosyl group and trifluoromethane sulfonyl group may be cited. They may be suitably selected from among the examples cited above.

### Steps 4 and 5

To the terminal of the metal-substituted alkyl group of the resultant compound (b), a nitrogen atom is introduced by the Gabriel reaction. Specifically, the compound (b) is caused to react with phthalimide potassium to obtain an adduct (c). The reaction is generally performed at a temperature in the range of from 0°C - the refluxing point of the solvent. As the solvent of this reaction is suitably selected from among dimethyl formamide, tetrahydrofuran, dimethyl sulfoxide, acetone, etc.

The compound (d) aimed at can be obtained by treating the produced adduct (c) with hydrous hydrazine. The reaction is performed at a temperature in the range of from 0°C to the refluxing point of the solvent. The solvent to be used in the reaction is suitably selected from among methanol, ethanol, dioxane, etc.

### Step 6

The halide (b) synthesized by the procedure consisting of Steps 1 - 3 in the absence or presence of a solvent is caused to react a primary or secondary amine at a temperature in the range of from room temperature to the refluxing point of the solvent. In this case, the solvent of the reaction can be suitably selected from among ethanol, methanol, tetrahydrofuran, etc. which do not participate in the reaction.

### Step 7

The amine (d) synthesized by the procedure consisting of Steps 1 - 5 in the absence or presence of a solvent is subjected to dehydration condensation with aldehyde to form a Schiff base. The solvent used herein can be selected from among benzene, toluene, ethanol, etc. A water separating device may be used where benzene or toluene is used as the solvent. The reaction temperature is in the range of from room temperature to the refluxing point of the solvent.

The produced Schiff base is reduced with boron sodium hydride in ethanol or methanol as a solvent to obtain an amine. This reduction is performed at a temperature in the range of from 0°C to room temperature.

### Examples

Now, this invention will be described more specifically below with reference to working examples. Of course, this invention is not limited to these working examples.

Prior to the working examples, typical methods for the production of raw material compounds for the compounds of this invention will be cited below as production examples.

### Production Example 1

### 2-(2,3-Dimethyl-4-fluorophenyl)aminobenzoic acid

A mixture of 3.3 g of 2-chlorobenzoic acid, 2.9 g of 2,3-dimethyl-4-fluoroaniline, 2.9 g of potassium carbonate, 200 mg of copper powder, and 200 ml of dimethyl formamide was stirred and refluxed for two hours. The resultant reaction solution was cooled and made to add water. The produced mixture was neutralized with concentrated hydrochloric acid and then extracted from ethyl acetate. The organic phase was separated, washed with saturated saline solution, dried with anhydrous magnesium sulfate, and thereafter subjected to vacuum distillation to expel the solvent. The residue of the vacuum distillation was refined by silica gel column chromatography to obtain 2.8 g of the captioned compound in the form of light yellow crystals.
¹H-NMR (400 MHz DMSO-d₆) δ
   9.32(s,1H),7.85(m,1H),7.26(m,1H),7.10(m,1H), 7.02(t,J=10.0,1H),6.65(t,J=7.0,1H),6.47(d,J=7.0,1H) 2.19(s,3H),2.08(s,3H)

### Production Example 2

### 2-Fluoro-3,4-dimethyl-9(10H)acridone

A mixture of 2.8 g of 2-(2,3-dimethyl-4-fluorophenyl)aminobenzoic acid obtained in Production Example 1 with 50 g of polyphosphoric acid was stirred at a temperature in the range of 70°C to 80°C for 30 minutes. The reaction mixture consequently obtained was combined with water to effect precipitation of crystals. The crystals were separated by filtration, washed with water, and dried to obtain 1.2 g of yellow crystals.
¹H-NMR 400 MHz (DMSO-d₆) δ
   10.53(s,1H),8.18(m,1H),7.90(d,J=10.0,1H),7.72-7.68(m,2H),7.23(t,J=8.0,1H),2.51(s,3H),2.31(d,J=2.0, 3H)

### Production Example 3

### Trifluoromethane sulfonic acid 3-bromopropyl ester

In 100 ml of dichloromethane, 6.2 g of 3-bromo-1-propanol and 3.8 g of pyridine were solved. The resultant solution was kept stirred at 0°C and 7.8 ml of trifluoromethane sulfonic anhydride was added meanwhile thereto and allowed to react therewith. The produced reaction solution was combined with water. The organic phase was separated, washed with water, dried with anhydrous magnesium sulfate, and thereafter subjected to vacuum distillation to expel the solvent and obtain 4.8 g of the captioned compound.
¹H-NMR 400 MHz(CDCl₂) δ
   4.71(t,J=6.0,2H),3.51(t,J=6.2,2H),2.36(m,2H)

### Production Example 4

### 2-Fluoro-3,4-dimethyl-10-(3-bromopropyl)-9-acridone

In 100 ml of tetrahydrofuran, 1.2 g of 2-fluoro-3,4-dimethyl-9(10H)acridone obtained in Production Example 2 was solved. To the produced solution, 3.7 ml of a hexane solution (1.6M) of n-butyl lithium was added dropwise. The resultant mixture was stirred at room temperature for 30 minutes. To this mixture, the trifluoromethane sulfonic acid 3-bromopropyl ether obtained in Production Example 3 was added dropwise. The produced mixture was stirred at room temperature for 30 minutes and then caused to add water. The mixture consequently obtained was extracted ethyl acetate. The organic phase was separated, washed with water, dried with anhydrous magnesium sulfate, and thereafter subjected to vacuum distillation to expel the solvent. The residue of the distillation was refined by silica gel column chromatography to obtain 310 mg of the captioned compound.
¹H-NMR 400 MHz(CDCl₂) δ
   8.33(m,1H),7.89(d,J=9.0,1H),7.66(m,1H), 7.57(d,J=8.0,1H),7.25(m,1H),4.45(t,J=7.0,2H), 2.90(t,J=7.0,2H),2.51(s,3H),2.34(d,J=2.0,3H) 1.80-1.72(m,2H)

### Production Example 5

### 2-Fluoro-3,4-dimethyl-10-(3-phthalimidepropyl)-9-acridone

A mixture obtained by adding 310 mg of the 2-fluoro-3,4-dimethyl-10-(3-bromopropyl)-9-acridone and 240 mg of phthalimide potassium to 50 ml of dimethyl formamide was stirred at 60°C for 20 minutes. The produced reaction solution was combined with water and the resultant mixture was extracted with ethyl acetate. The organic phase was separated, washed with water, dried with anhydrous magnesium sulfate, and thereafter subjected to vacuum distillation to expel the solvent. The residue was refined by silica gel column chromatography to obtain 280 mg of the captioned compound in the form of light yellow substance.
¹H-NMR (400 MHz CDCl₂) δ
   8.32(m,1H),7.81-7.68(m,5H),7.62(m,1H),7.49(d,J= 8.0,1H),7.23(m,1H),4.32(t,J=7.0,2H),3.38(t,J=7.0,2H), 2.44(s,3H),2.38(d,J=2.0,3H),1.64-1.58(m,2H)

### Production Example 6

### 3,4-Dimethyl-10-(3-bromopropyl)-9-acridone

To a solution of 9.0 g of 3,4-dimethyl-9(10H)-acridone in 300 ml of tetrahydrofuran which was kept stirred at 0°C, 25.2 ml of a 1.6M hexane solution of n-butyl lithium was added dropwise over a period of 15 minutes. The produced mixture was stirred at room temperature for 30 minutes and then cooled to 0°C. At 0°C, the trifluoromethane sulfonic acid 3-bromopropyl ether obtained in Production Example 3 was added dropwise. The resultant mixture was stirred at the same temperature for 30 minutes, then combined with water, and extracted with ethyl acetate. The organic phase was separated, washed with water, dried with anhydrous magnesium sulfate, and thereafter subjected to vacuum distillation. The residue was refined by silica gel column chromatography (15% ethyl acetate/85% hexane) to obtain 8.8 g of the captioned compound in the form of yellow oily substance.
¹H-NMR (400 MHz CDCl₂) δ
   1.76-1.84(m,2H), 2.44(s,3H), 2.50(s,3H), 2.91(t,J=6.0Hz,2H), 4.49(t,J=6.5,2H),7.19(d,J=8.0Hz,1H), 7.23-7.29(m,1H), 7.57(d,J=8.4Hz,1H), 7.64-7.70(m,1H), 8.18(d,J=8.0Hz,1H), 8.37(dd,J=2H)

### Production Example 7

### 3,4-Dimethyl-10-(3-phthalimidepropyl)-9-acridone

In 85 ml of N,N-dimethyl formamide, 5.8 g of the 3,4-dimethyl-10-(3-bromopropyl)-9-acridone and 3.8 g of phthalimide potassium salt were solved. The produced solution was heated to 60°C, stirred at the same temperature for 30 minutes, and then cooled to room temperature. The produced mixture was combined with water and then extracted from ethyl acetate. The organic phase was separated, washed with water, dried with anhydrous magnesium sulfate, and subjected to vacuum distillation to expel the solvent and induce precipitation of crystals. The crystals were separated by filtration to afford 5.2 g of the captioned compound in the form of light yellow crystals.
Melting point : 220-221°C
¹H-NMR (400 MHz CDCl₂) δ
   1.60-1.68(m,2H), 2.37(s,3H), 2.43(s,3H), 3.38(t,J=7.2HZ, 2H), 4.35(t,J=7.5Hz,2H), 7.10(d,J=8.0HZ,1H), 7.21-7.26 (m,1H), 7.51(d,J=8.4Hz,1H), 7.58-7.65(m,1H), 7.66-7.72 (m,2H), 7.74-7.79(m,2H), 8.12(d,J=8.0HZ,1H), 8.35(dd,J=1.6Hz,8.4Hz,1H)

### Example 1

### 2-Fluoro-3,4-dimethyl-10-(3-aminopropyl)-9-acridone

In 50 ml of methanol, 280 mg of the 2-fluoro-3,4-dimethyl-10-(3-phthalimidepropyl)-9-acridone obtained in Production Example 5 and 1.0 g of hydrazine hydrate were solved. The produced solution was stirred and refluxed for 30 minutes. The resultant reaction solution was cooled to room temperature, alkalinized with an aqueous 1N sodium hydroxide solution, and extracted from ethyl acetate. The organic phase was separated, washed with water, dried with anhydrous magnesium sulfate, and thereafter subjected to vacuum distillation to expel the solvent. The residue was refined by silica gel column chromatography to obtain 180 mg of the captioned compound in the form of a yellow oily substance.
¹H-NMR (400 MHz CDCl₂) δ
   8.34(dd,J=1.6Hz,8.0Hz,1H),7.89(d,J=9.4Hz,1H)),7.66(m, 1H),7.57(d,J=8.4Hz,1H),7.24(m,1H),4.35(t,J=7.0Hz,2H), 2.50(s,3H),2.34(d,J=2.0Hz,3H),2.23(t,J=7.0Hz,2H), 1.40-1.33(m,2H)

### Example 2

### 3,4-Dimethyl-10-(3-aminopropyl)-9-acridone

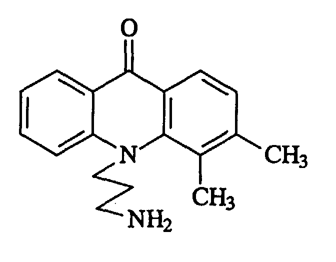

In 100 ml of methanol, 5.2 g of the 3,4-dimethyl-10-(3-phthalimidepropyl)-9-acridone obtained in Production Example 7 and 5.0 g of hydrazine hydride were suspended. The produced suspension was stirred and refluxed for 30 minutes. The resultant reaction solution was cooled to room temperature, alkalinized with an aqueous 1N sodium hydroxide solution, and extracted from ethyl acetate. The organic phase was separated, washed with water, dried with anhydrous magnesium sulfate, and then concentrated under reduced pressure. The residue was refined by silica gel column chromatography (10% methanol/90% dichloromethane) to obtain 3.76 g of the captioned compound in the form of a yellow oily substance.
¹H-NMR (400 MHz CDCl₂) δ
   1.35-1.45(m,2H), 2.15-2.25(m,2H), 2.43(s,3H), 2.48(s,3H), 4.39(t,J=7.0Hz,2H), 7.17(d,J=8.0Hz,1H), 7.22-7.28(m,1H), 7.58(d,J=8.4Hz,1H), 7.62-7.68(m,1H), 8.18(d,J=8.0Hz,1H), 8.36(dd,J=1.6Hz,8.4Hz,1H)

### Example 3

### 3,4-Dimethyl-10-(3-aminopropyl)-9-acridone oxalate

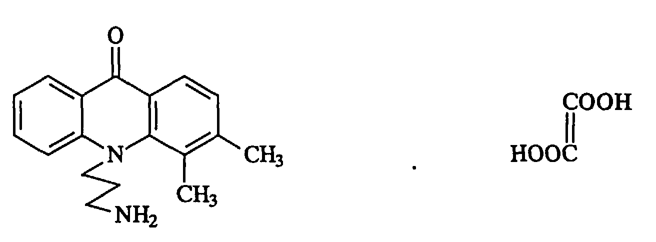

To a solution of 3.76 g of the 3,4-dimethyl-10-(3-aminopropyl)-9-acridone in 30 ml of ethanol, a solution of 1.69 g of oxalic acid in 20 ml of ethanol was added as kept stirred at room temperature. They were stirred together at the same temperature for 30 minutes. The resultant solution was combined with diethyl ether to induce precipitation of crystals. The crystals were separated by filtration to obtain 3.97 g of captioned compound in the form of yellow crystals.
Melting point: 200 - 201°C
¹H-NMR (400 MHz CDCl₂) δ
   1.34-1.44(m,2H), 2.35-2.43(m,2H), 2.39(s,3H), 2.44(s,3H), 4.40(t,J=7.2Hz,2H), 7.22(d,J=8.0Hz,1H), 7.25-7.31(m,1H), 7.72-7.78(m,1H), 7.84(d,J=8.0Hz,1H), 7.95(d,J=8.0Hz,1H), 8.14(dd,J=1.6Hz,8.4Hz,1H)

The compounds enumerated in Tables 1-3 were synthesized by following the procedure of Examples 1-3 with necessary modifications.

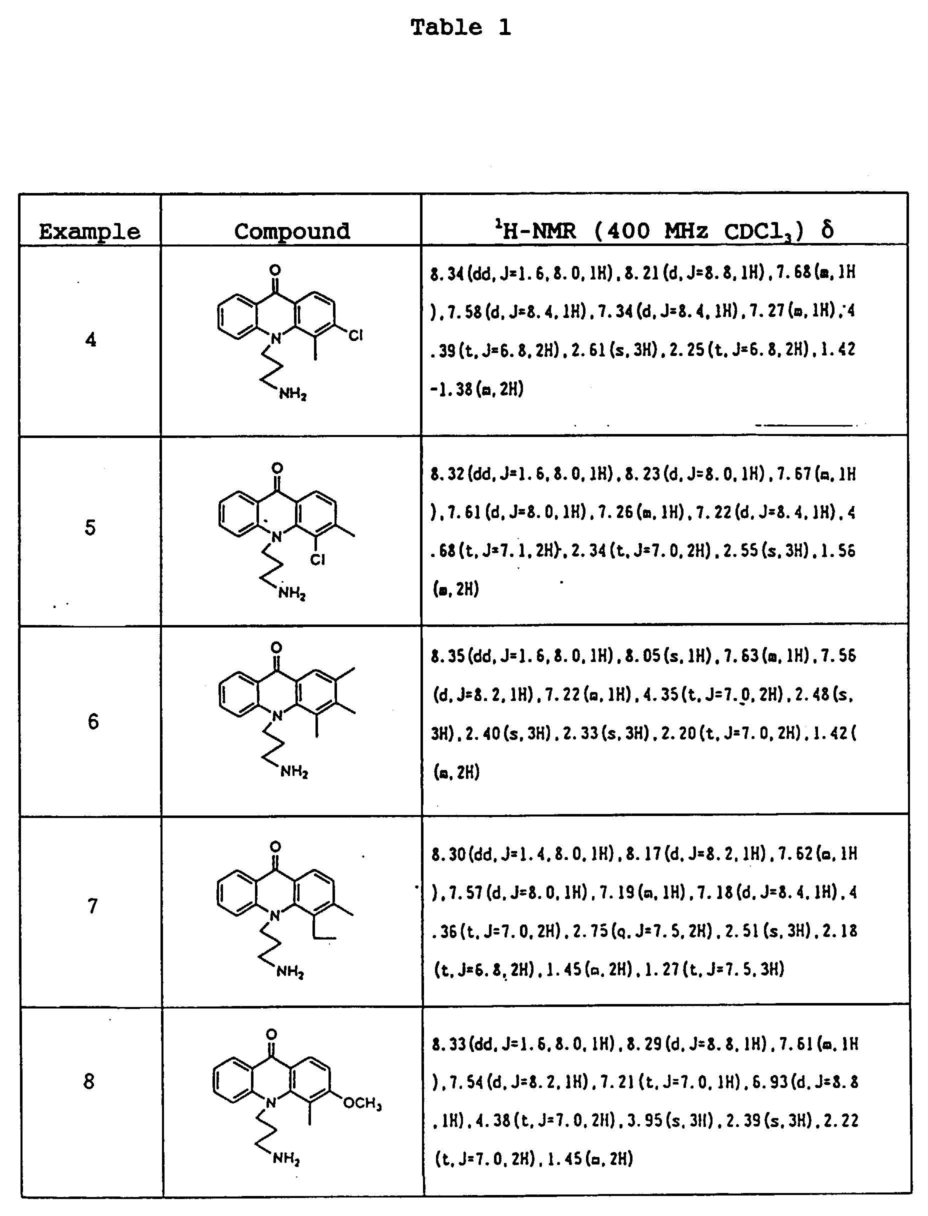

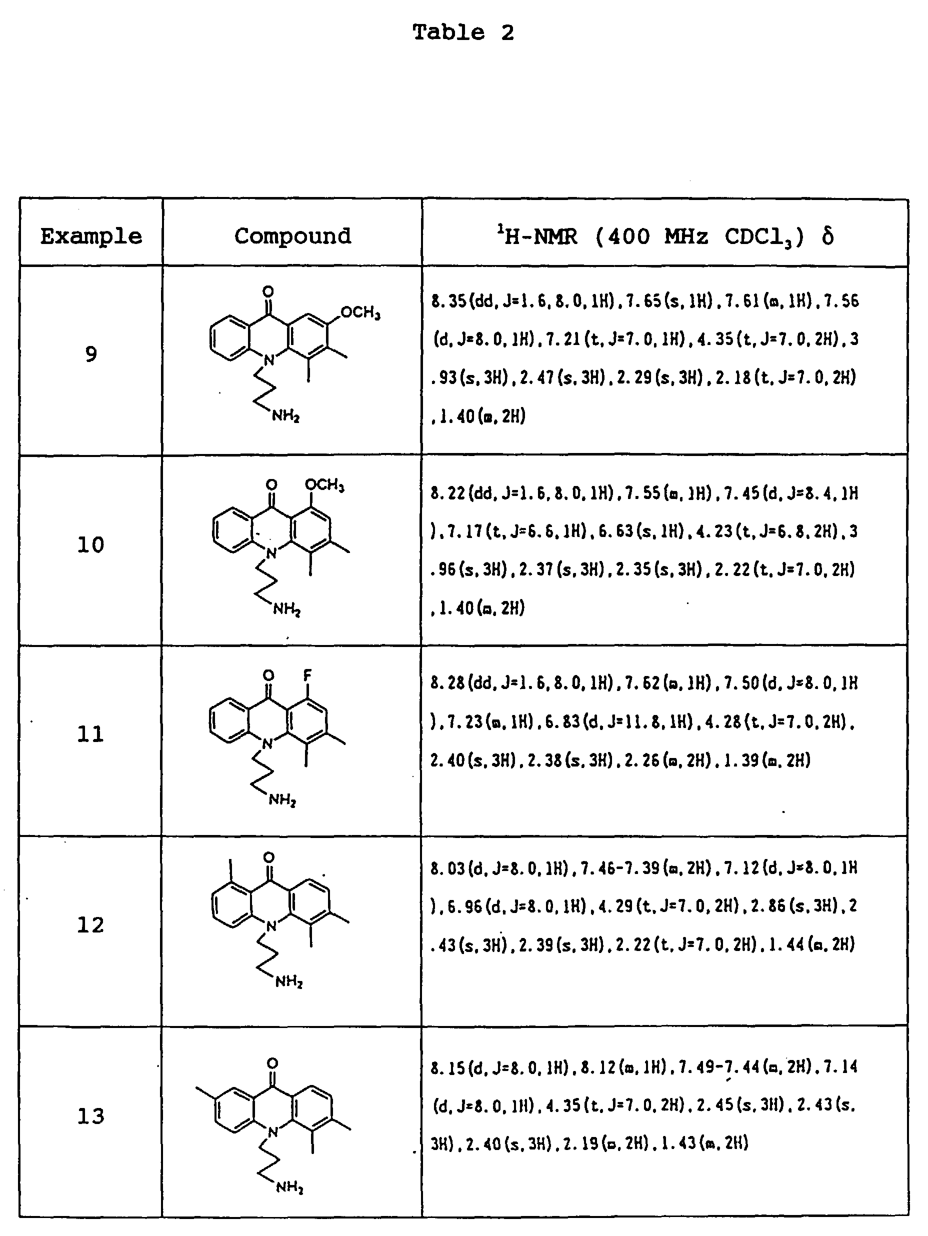

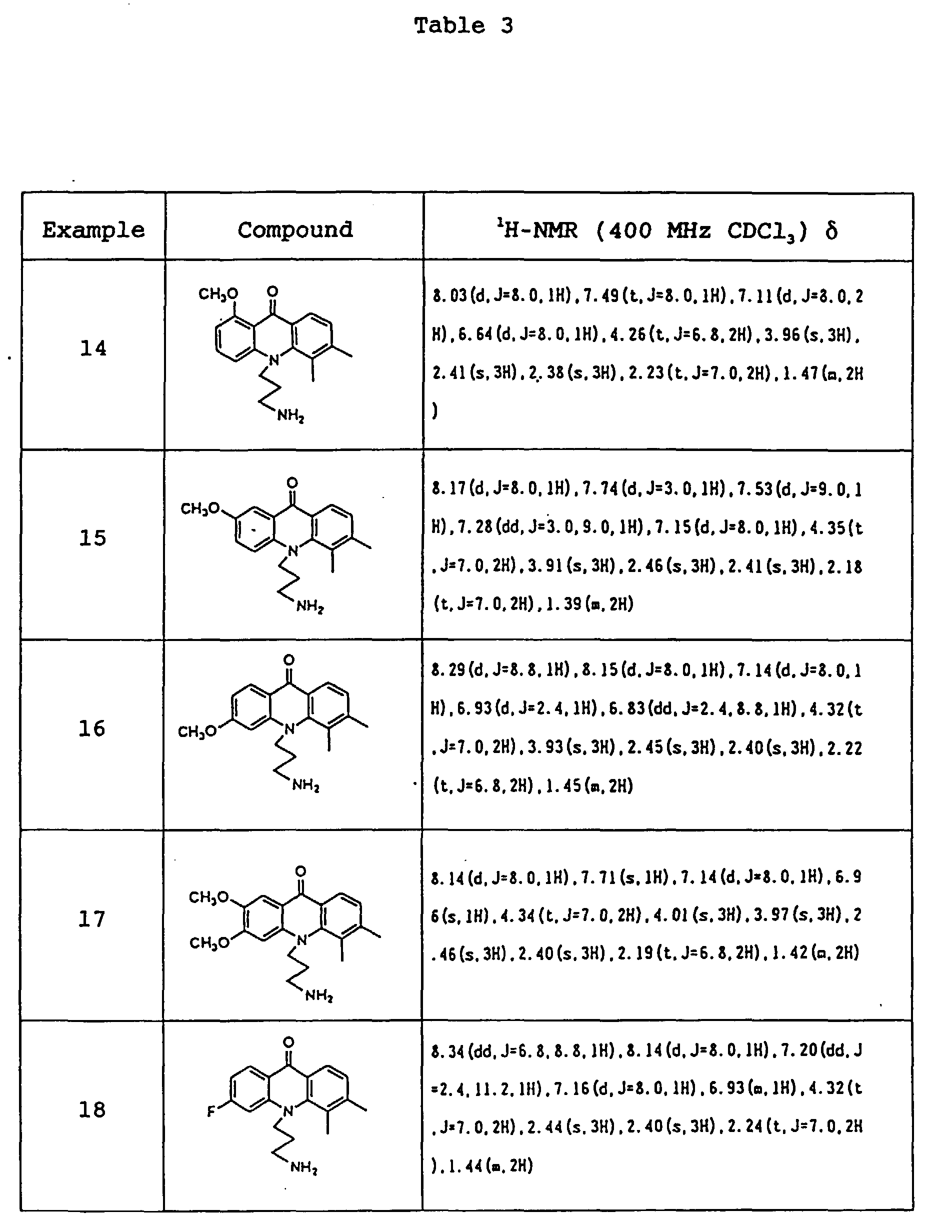

### Example 19

### 3,4-Dimethyl-10-(3-benzylaminopropyl)-9-acridone

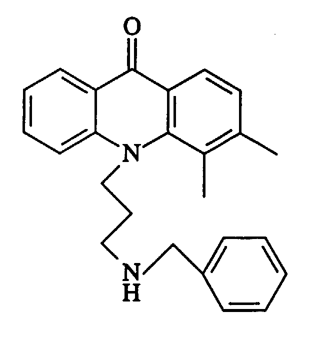

In 20 ml of ethanol, 350 mg of the 3,4-dimethyl-10-(3-bromopropyl)-9-acridone synthesized by following the procedure of Production Example 1 - 4 with necessary modification and 535 mg of benzyl amine were dissolved. The produced solution was starred and refluxed for five hours. The resultant solution was combined with an 0.5-N sodium hydroxide solution and then extracted from ethyl acetate. The organic phase was separated, washed with saturated saline solution, dried with anhydrous magnesium sulfate, and thereafter subjected to vacuum distillation to expel the solvent. The residue was refined by column chromatography to obtain 354 mg of the captioned compound.
¹H-NMR (400 MHz CDCl₂) δ
   8.37(dd,J=8.4,1.6Hz,1H),8.19(d,J=8.4,1H),7.64(m,1H), 7.57(d,J=8.8,1H),7.28-7.18(m,4H),7.16(d,J=7.6,1H), 7.60-7.30(m,2H),4.42(t,J=6.8,2H),3.39(s,2H), 2.46(s,3H),2.40(s,3H),2.13(t,J=6.4,2H),1.45-1.40(m,2H)

The compounds represented by the following general formula and enumerated in Tables 4 - 6 were synthesized in a similar manner to the procedure of Example 19.

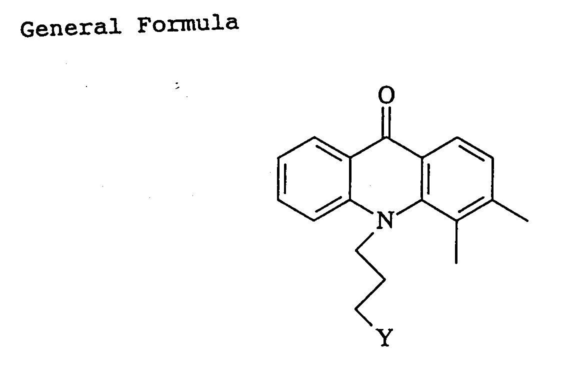

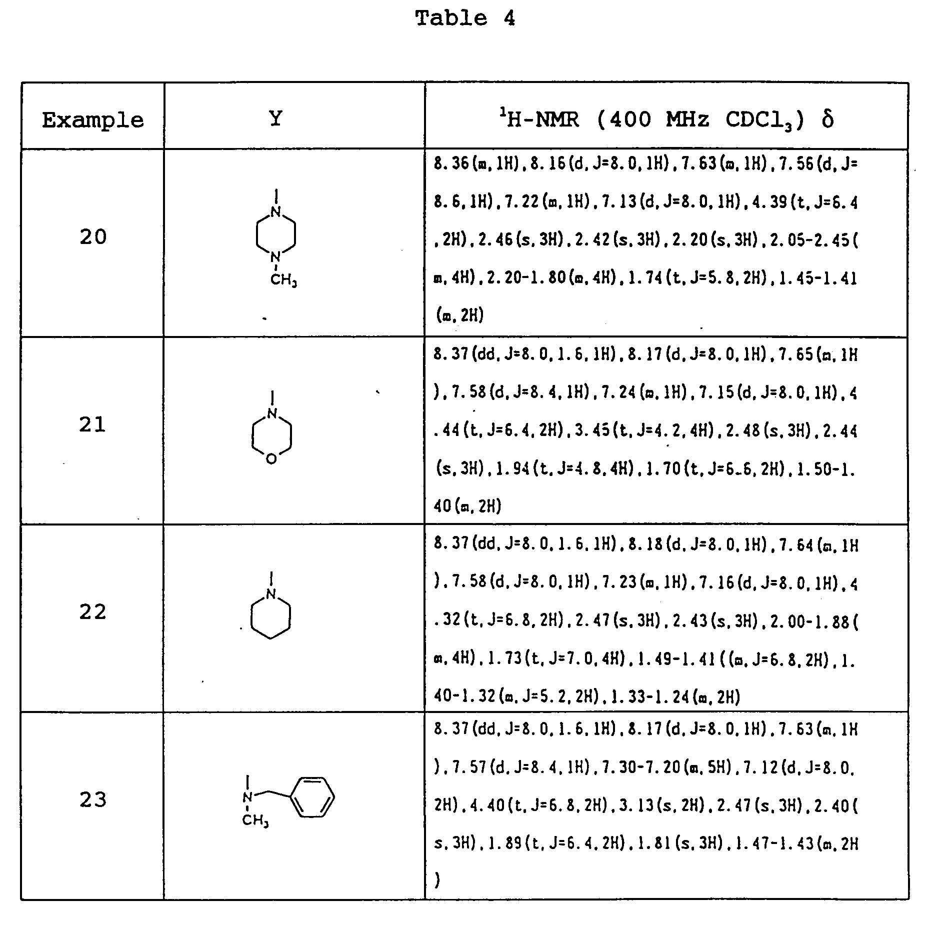

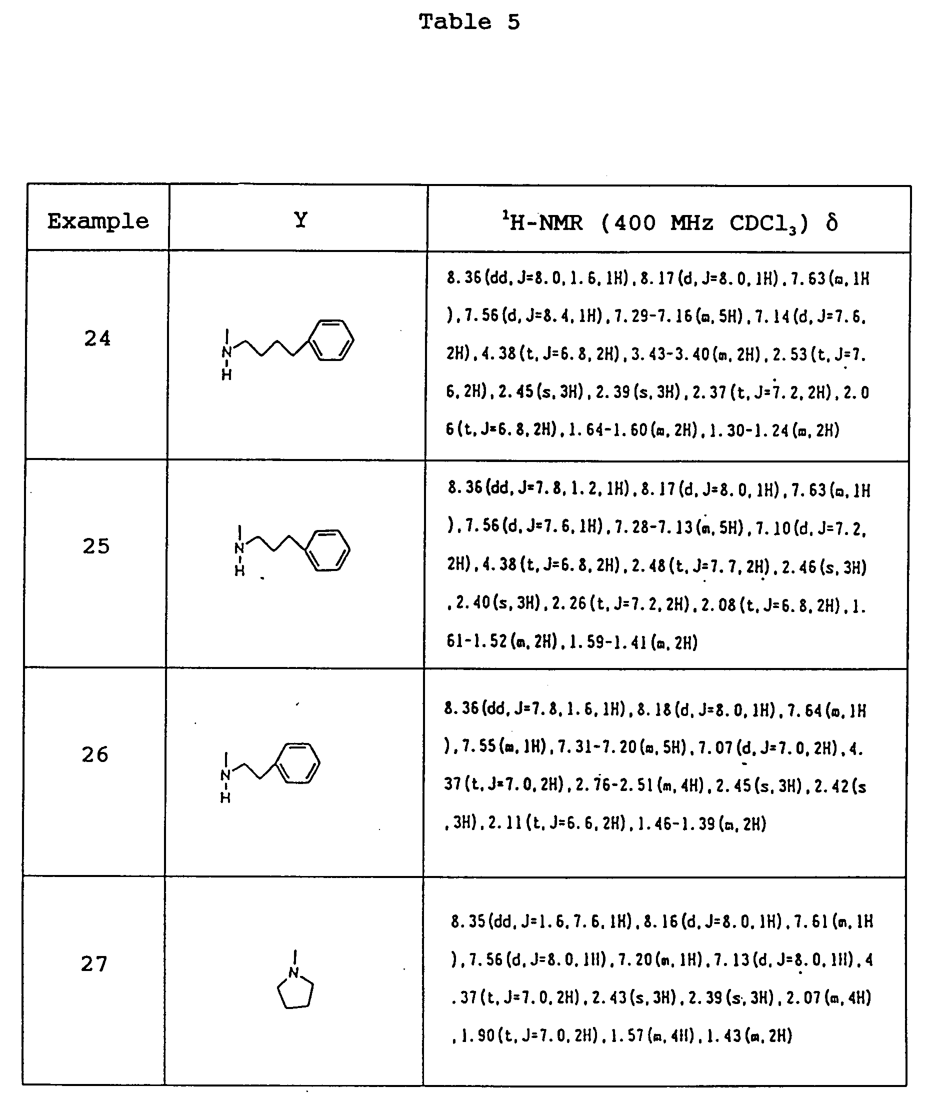

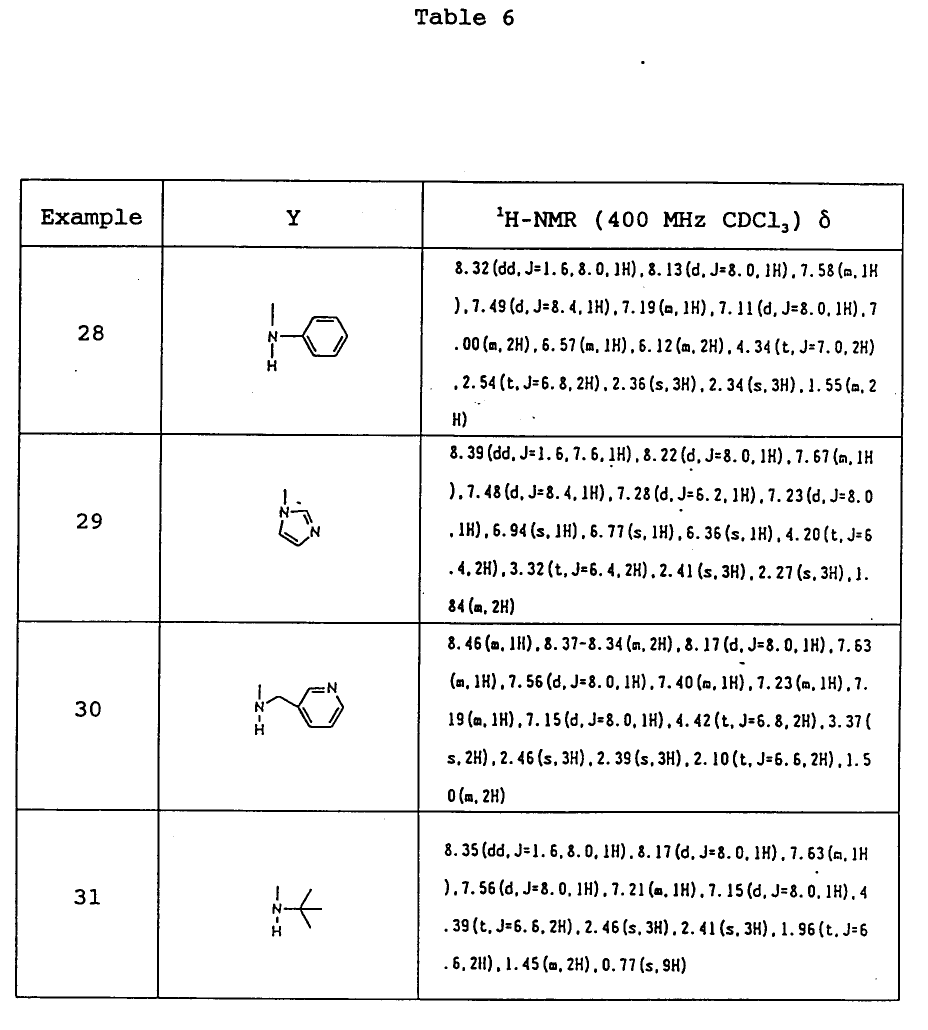

### Example 32

### 3,4-Dimethyl-10-[3-(2-imidazoylmethyl)aminopropyl]-9 acridone

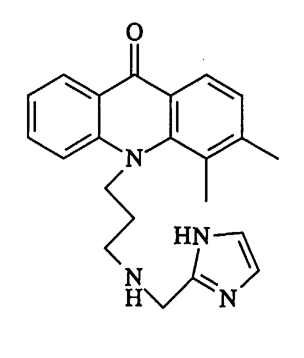

In toluene, 350 mg of the 3,4-dimethyl-10-(3-aminopropyl)-9-acridone synthesized by following the procedure of Example 2 and 132 mg of imidazol-2-aldehyde were solved. The produced solution was stirred and refluxed for two hours by the use of a refluxing device provided with a dehydrater. The reaction solution was cooled and then mixed with methanol and subsequently with 150 mg of boron sodium hydride. The produced mixture was stirred for 20 minutes. The resultant reaction solution was neutralized with tartaric acid and then subjected to vacuum evaporation to remove the solvent. The residue was refined by reversed-phase silica gel column chromatography to obtain 40 mg of the captioned compound.
¹H-NMR (400 MHz DMSO-d₆) δ
   8.12(dd,J=1.6Hz,8.0Hz,1H),7.94(d,J=8.0Hz,1H),7.82(d, J=8.4Hz,1H),7.71(m,1H),7.25(m,1H),7.19(d,J=8.0Hz,1H), 6.82(s,2H),4.38(t,J=7.0Hz,2H),3.39(s,2H),2.42(s,3H), 2.37(s,3H),2.07-2.06(m,2H),1.38-1.20(m,2H)

The compounds represented by the following general formula and enumerated in Table 7 and Table 8 were synthesized in a similar manner to the procedure of Example 32, while changing the species of aldehyde.

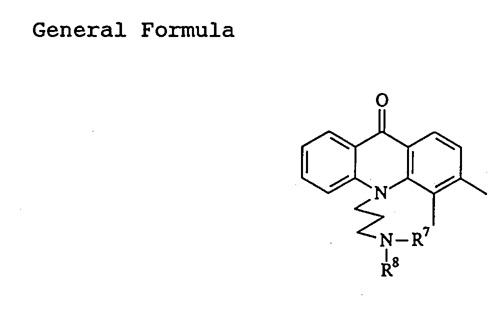

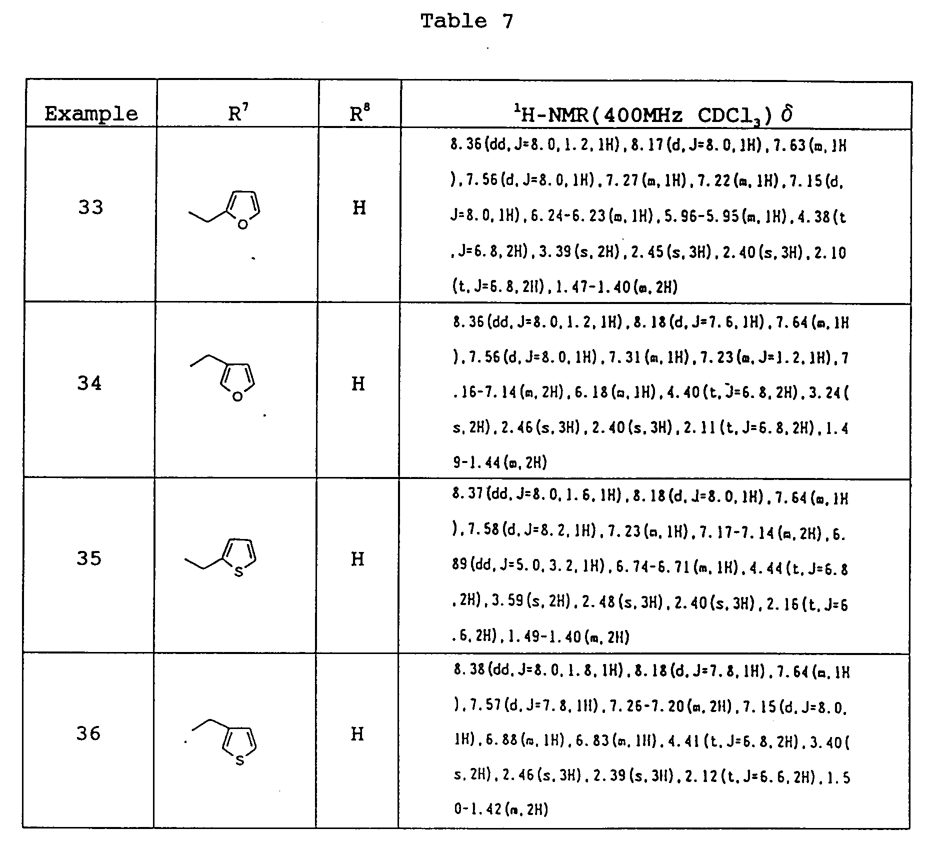

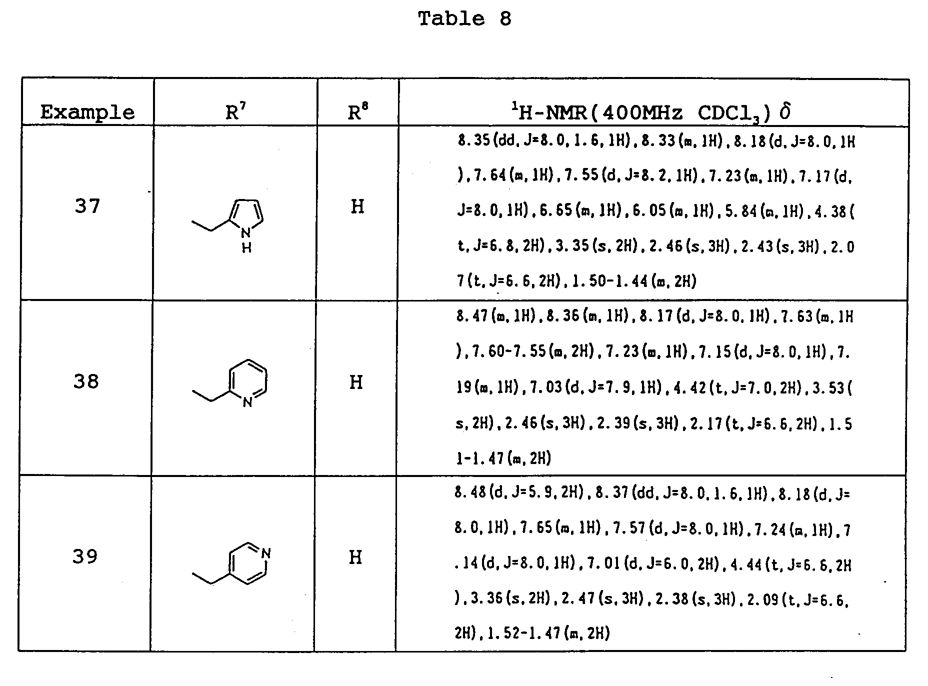

A pharmacological test example will be cited below for the purpose of describing in detail the outstanding effect of the compounds of this invention.

### [Study on activity to repress the liberation of various mediators from rat basophilic leukemic cell strain (RBL-2H3) and activity to repress thyrosine kinase]

The cells of RBL-2H3, one species of rat cell line, carry out not only the liberation of histamine and serotonin but also the formation and liberation of prostaglandins, TNFα, etc., i.e. inflammatory mediators, owing to the specific antigen of the IgE antibody bound to the IgE receptor. In the present test system, therefore, the cells were marked with serotonin and arachidonic acid labeled in advance with [³H] and cultured in the presence of a compound under test and in the absence thereof and the amounts of the mediators liberated in the culture medium were measured. Thus, the given compounds were tested for activity to repress the liberation of various mediators. The TNFα was determined by means of bioassay.

The results of the test of the compounds for the activity to repress the liberation of serotonin are shown in Table 9.

**Table 9**

| Compound | | IC₅₀ (µM) in liberation of serotonin from RBL-2H3 cells |
|---|---|---|
| Example | 1 | 1.7 |
| | 2 | 6 |
| | 3 | 12 |
| | 4 | 23 |
| | 12 | 15 |
| | 13 | 10 |
| | 14 | 5 |
| | 15 | 8 |
| | 16 | 8 |
| | 17 | 20 |
| | 18 | 6 |
| | 19 | 3 |
| | 21 | 8 |
| | 22 | 8 |
| | 23 | 0.6 |
| | 24 | 7 |
| | 25 | 9 |
| | 26 | 15 |
| | 27 | 3 |
| | 30 | 12 |
| | 31 | 3 |
| | 32 | 0.6 |
| | 33 | 2.4 |
| | 34 | 3 |
| | 35 | 1.2 |
| | 36 | 1.2 |
| | 37 | 0.1 |
| | 38 | 0.3 |
| | 39 | 2.0 |

### IC₅₀ (µM) in liberation of serotonin from RBL-2H3 cells

It has been demonstrated immunologically that in the system for transfer of information from the IgE receptor, when the antigen is bound to the IgE antibody which has been bound to the IgE receptor, the thyrosine activating motif area of the IgE receptor γ chain is phosphorylated and the 72 kDa thyrosine kinase is bound to the area and consequently activated. The question as to whether or not the compound under test would repress the bondage of the 72 kDa thyrosine kinase to the thyrosine activating motif area was studied by using the peptide segment of a thyrosine activating motif area, an antiphospho-thyrosine antibody, etc. For the purpose of confirming the selectivity of the compound under test with respect to the effect of repressing thyrosine kinase, the effectiveness of the compound in repressing other types of thyrosine kinases was investigated by using Jurkat cells (T-cells) and NIH3T3 cells (fibroblast).

### (Test results and discussion)

The compounds of this invention, as shown in Table 9, repressed the liberation of serotonin, one of the mediators, from the RBL-2H3 cells.

The compound obtained by the procedure of Example 3, at an IC₅₀ value of 12 µM, repressed the liberation of histamine, serotonin, and arachidonic acid from the RBL-2H3 cells due to the antigenic stimulus and, at the same concentration, repressed the formation and liberation of TNFα. It has been also demonstrated immunologically that this compound, at an IC₅₀ value of 12 µM, repressed the activation of the 72 kDa thyrosine kinase by the antigenic stimulus and also repressed, at the same concentration, the activation of phospholipase C, one of the intracellular substrates of this thyrosine kinase. It has been confirmed that this compound repressed the bondage of the 72 kDa thyrosine kinase to the peptide in the phosphorylated thyrosine activating motif area of the human type IgE receptor γ chain at an IC₅₀ value of 30 µM.

These observations clearly indicate that the compounds of this invention inhibit the activation of the 72 kDa thyrosine kinase and consequently manifest an activity to repress the liberation of such mediators as mentioned above owing to the inhibition of the bondage of the IgE receptor γ chain to the 72 kDa thyrosine kinase.

Concerning the selectivity with respect to the thyrosine kinase, the compound of Example 3 manifested absolutely no activity to repress the antigenic stimulus in the Jurkat cells, a fact which clearly indicates that this compound shows no activity to repress such thyrosine kinases as p561ck and fyn. By a test using the NIH3T3 cells, it has been immunologically demonstrated that this compound shows no activity to repress the activation of the receptor type thyrosine kinase relative to such growth factors as FGF, PDGF, and EGF.

From the results shown above, it is clear that the compounds contemplated by this invention are useful as preventive·curative agents for asthma, allergic rhinitis, atopic dermatitis, urticaria, valerian fever, digestive tract allergy, and food allergy.

## Claims

1. An acridone derivative represented by the following formula (I) or a pharmacologically acceptable salt thereof: 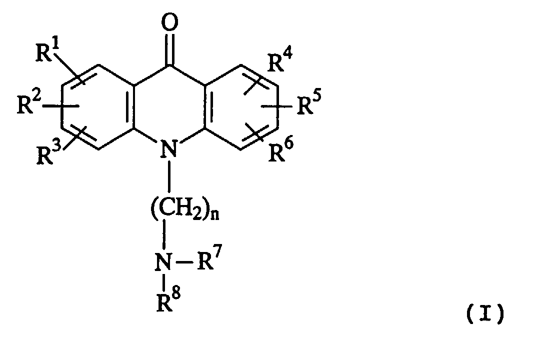 (wherein R¹, R², R³, R⁴, R⁵, and R⁶ invariably or variably represent a hydrogen atom, a lower alkyl group, a lower alkoxy group, or a halogen atom, R⁷ and R⁸ invariably or variably represent a hydrogen atom, a lower alkyl group, an aryl group, a heteroaryl group, an aralkyl group, or a heteroaryl alkyl group, providing R⁷ and R⁸, when taken together with a nitrogen atom to which they are bound, form a ring of five or six members, wherein said ring optionally possesses as component atoms thereof an oxygen atom or a sulfur atom or a group represented by the formula: >N-R⁹ (wherein R⁹ represents a hydrogen atom, a lower alkyl group, an aryl group, or an aralkyl group) in addition to carbon atoms, and n represents an integer in the range of 1 - 6).

2. A preventive·curative agent for diseases in which the thyrosine kinase inhibiting action is effective, which agent has as an active component thereof an acridone derivative represented by the formula (I) and/or a pharmacologically acceptable salt thereof: 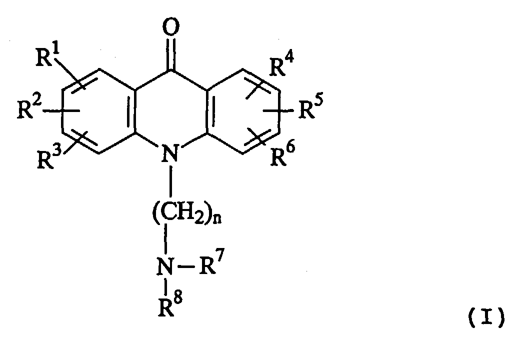 (wherein R¹, R², R³, R⁴, R⁵, and R⁶ invariably or variably represent a hydrogen atom, a lower alkyl group, a lower alkoxy group, or a halogen atom, R⁷ and R⁸ invariably or variably represent a hydrogen atom, a lower alkyl group, an aryl group, a heteroaryl group, an aralkyl group, or a heteroaryl alkyl group, providing R⁷ and R⁸, when taken together with a nitrogen atom to which they are bound, form a ring of five or six members, wherein said ring optionally possesses as component atoms thereof an oxygen atom or a sulfur atom or a group represented by the formula: >N-R⁹ (wherein R⁹ represents a hydrogen atom, a lower alkyl group, an aryl group, or an aralkyl group) in addition to carbon atoms, and n represents an integer in the range of 1 - 6).

3. A preventive·curative agent according to claim 2, wherein said disease in which the thyrosine kinase inhibiting action is effective is a disease in which the antiallergic action is effective.

4. A preventive-curative agent for allergic diseases, having as an active component thereof an acridone derivative represented by the formula (I) and/or a pharmacologically acceptable salt thereof: 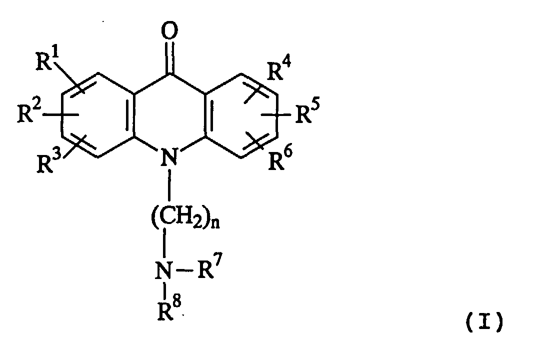 (wherein R¹, R², R³, R⁴, R⁵, and R⁶ invariably or variably represent a hydrogen atom, a lower alkyl group, a lower alkoxy group, or a halogen atom, R⁷ and R⁸ invariably or variably represent a hydrogen atom, a lower alkyl group, an aryl group, a heteroaryl group, an aralkyl group, or a heteroaryl alkyl group, providing R⁷ and R⁸, when taken together with a nitrogen atom to which they are bound, form a ring of five or six members, wherein said ring optionally possesses as component atoms thereof an oxygen atom or a sulfur atom or a group represented by the formula: >N-R⁹ (wherein R⁹ represents a hydrogen atom, a lower alkyl group, an aryl group, or an aralkyl group) in addition to carbon atoms, and n represents an integer in the range of 1 - 6).

5. A preventive·curative agent according to claim 4, wherein said allergic diseases are asthma, allergic rhinitis, atopic dermatitis, urticaria, valerian fever, digestive tract allergy, or food allergy.

6. Use of an acridone derivative according to claim 1 for the preperation of a pharmaceutical composition for the treatment of allergic diseases selected from asthma, allergic rhinitis, atopic dermatitis, urticaria, valerian fever, digestive tract allergy, food allergy and the like.
